Europäisches Patentamt

European Patent Office

Office européen des brevets

(11) Veröffentlichungsnummer: **0 224 886**
**A1**

## (12) EUROPÄISCHE PATENTANMELDUNG

(21) Anmeldenummer: **86116577.7**

(22) Anmeldetag: **28.11.86**

(51) Int. Cl.⁴: **A 61 B 6/00**

(30) Priorität: **29.11.85 DE 3542333**

(43) Veröffentlichungstag der Anmeldung:
**10.06.87 Patentblatt 87/24**

(84) Benannte Vertragsstaaten:
**DE FR GB**

(71) Anmelder: **Siemens Aktiengesellschaft Berlin und München**
**Wittelsbacherplatz 2**
**D-8000 München 2(DE)**

(72) Erfinder: **Barud, Sigvard**
**Roedluvevaegen 73**
**S-17570 Jaerfaella(SE)**

(54) **Röntgenuntersuchungsgerät.**

(57) Röntgenuntersuchungsgerät mit einer Säule (4) und einer Halterung (6), die als ein zweiteiliger Gelenkarm ausgebildet ist, die in Bezug auf die Säule in vertikaler Richtung verstellbar ist und einen gebogenen Träger (7) hält. An dem einen Ende des Trägers ist eine Strahlungsquelle (12) und an dessen anderem Ende ein Bildschichtträger (13) aufeinander ausgerichtet befestigt. Der Träger ist um eine erste Welle (15) drehbar gelagert und um eine weitere, horizontale und senkrecht zur ersten Welle am vorderen Ende der Halterung liegende zweite Welle (8) schwenkbar gelagert. Für jede der Translations- und Drehbewegungen ist ein Antrieb vorgesehen (17,19,21,23,25,27). Damit das Röntgenuntersuchungsgerät in mehreren Koordinaten in Bezug auf den Untersuchungstisch bewegbar ist, wird vorgeschlagen, dass die Säule (4) um eine vertikale Achse (5) drehbar gelagert ist und die gemeinsame Schwenkachse (9) des zweiteiligen Gelenkarmes (6) etwa vertikal verläuft.

FIG 1

EP 0 224 886 A1

0224886

Siemens Aktiengesellschaft
Berlin und München

Unser Zeichen
VPA 85 P 7315 E

## Röntgenuntersuchungsgerät

Die Erfindung betrifft ein Röntgenuntersuchungsgerät mit einer Säule und einer Halterung, die als ein zweiteiliger Gelenkarm ausgebildet ist, die in Bezug auf die Säule in vertikaler Richtung verstellbar ist und einen gebogenen Träger hält, an dessen einem Ende eine Strahlungsquelle und an dessen anderem Ende ein Bildschichtträger aufeinander ausgerichtet befestigt sind, und wobei der Träger um eine erste Welle drehbar gelagert und um eine weitere, horizontale und senkrecht zur ersten Welle am vorderen Ende der Halterung liegende zweite Welle schwenkbar gelagert ist und für jede der Translations- und Drehbewegungen ein Antrieb vorsehbar ist.

Durch die deutsche Patentanmeldung P 34 13 348.8 ist ein Röntgenuntersuchungsgerät dieser Art mit einer Säule bekannt, die parallel zur Längsseite eines ortsfesten Untersuchungstisches fahrbar ist. Die an der Säule angebrachte Halterung ist entweder durch den zweiteiligen Gelenkarm, dessen gemeinsame Schwenkachse horizontal verläuft, oder durch eine Teleskopsäule in ihre Längsrichtung einstellbar. Mit diesem Röntgenuntersuchungsgerät können Einebeneuntersuchungen eines auf dem Untersuchungstisch ruhenden Patienten vorgenommen werden. Das Röntgenuntersuchungsgerät ist also immer, unabhängig von der Lage des Untersuchungstisches, lediglich in einer Tischkoordinate bewegbar, d.h. dass die Halterung lediglich seitlich und entlang dem Patient bewegt werden kann.

Der Erfindung liegt die Aufgabe zugrunde, ein Röntgenunter-

Lst 1 Ed / 19.11.1985

suchungsgerät der eingangs genannten Art zu schaffen, das in mehreren Koordinaten in Bezug auf den Untersuchungstisch bzw. Patienten bewegbar ist.

Diese Aufgabe ist erfindungsgemäss dadurch gelöst, dass die Säule um eine vertikale Achse drehbar gelagert ist und die gemeinsame Schwenkachse des zweiteiligen Gelenkarmes etwa vertikal verläuft. Durch die zusätzlichen Bewegungsmöglichkeiten des Röntgenuntersuchungsgerätes weist der gebogene Träger mit der Röntgenröhre und der Bildschichtträger gegenüber dem Stand der Technik grössere Einstellungsmöglichkeiten auf. Hierdurch kann das Röntgenuntersuchungsgerät bzw. die Halterung so angesteuert werden, dass der Teil des Gelenkarmes, der den Träger hält, parallel zur Längsrichtung des Untersuchungstisches verläuft. Das Röntgenuntersuchungsgerät kann dadurch für sowohl Einebene- als auch für Zweiebenenuntersuchungen verwendet werden. Zweiebenenuntersuchungen werden häufig bei Herz- und Schädeluntersuchungen benutzt.

Bei einer Zweiebenenuntersuchung werden zwei Röntgenuntersuchungsgeräte, ein Bodengerät und häufig ein deckenaufgehängtes Gerät, verwendet. Die Röntgenröhre bzw. Bildschichtträger der beiden Geräte werden so aufeinander ausgerichtet, dass der zu untersuchende Abschnitt gleichzeitig von zwei Seiten aufgenommen wird. Das deckenaufgehängte Gerät weist einen Träger für die Röntgenröhre bzw. Bildschichtträger auf, der grösser als der Träger des Bodengerätes ist und diesen umgreift. Er ist dabei quer zur Längsrichtung des Untersuchungstisches angeordnet.

In einer vorteilhaften Weiterbildung der Erfindung wird vorgeschlagen, dass der von der Säule ausgehende Teil des Gelenkarmes als Parallelogrammarm ausgebildet ist. Dadurch

0224886
VPA 85 P 7315 E

ist erreicht, dass die gemeinsame Schwenkachse des zweiteiligen Gelenkarmes in jeder Lage der Gelenkarme vertikal verläuft, so dass der Gelenkarm, der den Träger hält, bei einer vertikalen Bewegung nicht um seine eigene Achse gedreht wird. Dies hat den Vorteil, dass der Träger beim Heben und Senken eine Parallelbewegung durchführt.

Die Erfindung ist nachfolgend anhand eines in den Zeichnungen dargestellten Ausführungsbeispieles näher erläutert. Es zeigen:

Fig. 1 ein Röntgenuntersuchungsgerät nach der Erfindung

Fig. 2 eine schematische Darstellung einer Steuervorrichtung für ein Röntgenuntersuchungsgerät nach Fig. 1 und

Fig. 3 eine Draufsicht auf ein Röntgenuntersuchungsgerät nach Fig. 1

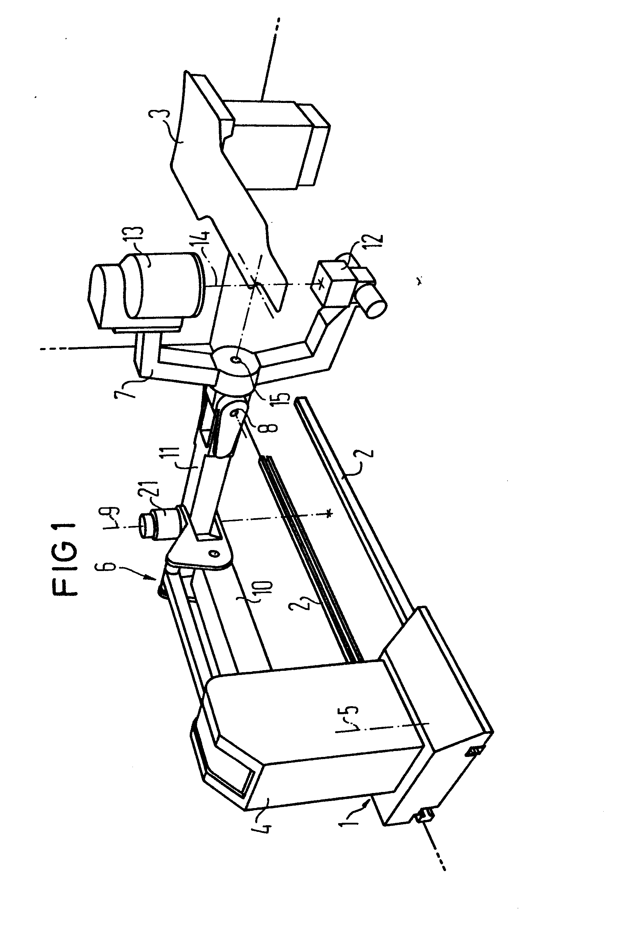

Fig. 1 zeigt ein Röntgenuntersuchungsgerät mit einem Stativwagen 1, der auf Bodenschienen 2 längsverschiebbar ist und parallel zu einem ortsfesten Patientenlagerungstisch 3 geführt werden kann.

Der Stativwagen 1 weist eine Säule 4 auf, die um eine vertikale Achse 5 drehbar gelagert ist. An der Säule 4 ist eine Halterung 6 für einen u- oder v-förmig gebogenen Träger 7 vertikal einstellbar gelagert. Die Halterung 6 ist als ein zweiteiliger Gelenkarm ausgebildet, bei dem die gemeinsame Schwenkachse 9 etwa vertikal verläuft. Der von der Säule 4 ausgehende erste Teil 10 des Gelenkarmes 6 ist als Parallelogrammarm ausgebildet. Der Träger 7 ist um eine senkrecht zur Längsrichtung des zweiten Teiles 11 des Gelenkarmes 6 an dessen vorderen Ende angebrachten Welle 8 schwenkbar gelagert. An dem einen Ende des Trägers 7 ist eine Röntgenröhre 12 und an dem anderen Ende ein Bildschichtträger 13 befestigt. Die Röntgenröhre 12 ist mit ihrem Zentralstrahl 14 auf den Bildschichtträger 13 zentriert. Der Träger 7 ist ferner um eine Welle 15 des zweiten Teiles 11 des Gelenkarmes drehbar gelagert.

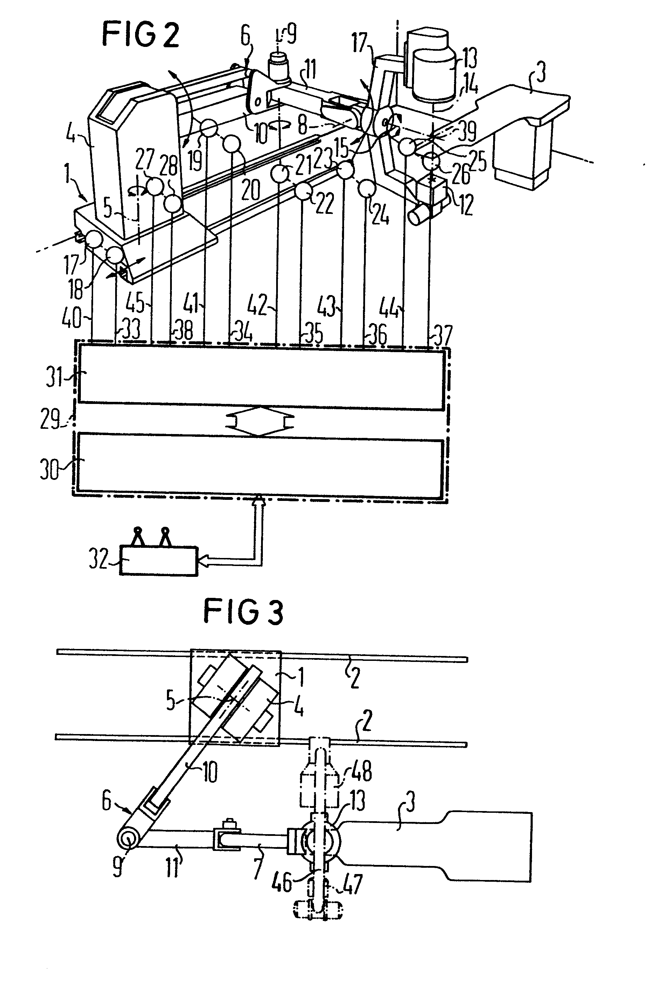

In Fig. 2 ist dargestellt, dass für jede Verstell-, Dreh- oder Schwenkbewegung der Geräteteile ein Antrieb und ein Lagesensor vorgesehen sind. So wird der Stativwagen 1 mittels eines Motors 17 und eines Lagesensors 18 gesteuert. Die Höhenverstellung der Halterung 6 erfolgt mittels eines Motors 19 und eines Lagesensors 20 und die Schwenkung des zweiteiligen Gelenkarmes 6 um die Achse 9 wird durch einen Motor 21 und einen Lagesonsor 22 geregelt. Ein weiterer Motor 23 und ein Lagesensor 24 steuern die Schwenkbewegung des Trägers 7 um die Welle 8. Ferner ist ein Motor 25 und ein Lagesensor 26 für die Drehung des Trägers 7 um die Welle 15 vorgesehen. Schliesslich ist für die Drehung der Säule 4 um die vertikale Achse 5 ein Motor 27 und ein Lagesensor 28 zugeordnet.

Als Regeleinrichtung 29 für die Motoren 17,19,21,23,25,27 und die Lagesensoren 18,20,22,24,26,28 dient ein Computer 30 und eine Steuervorrichtung 31. An der Regeleinrichtung 29 ist ein Steuerpult 32 angeschlossen, wodurch die Lage der Röntgenröhre 12 bzw. des Bildschichtträgers 13 gesteuert werden. Eine derartige Regeleinrichtung mit Steuerpult ist an sich grundsätzlich durch die US PS 4,019,059 bekannt und wird daher nicht näher beschrieben.

Wenn eine Lageänderung der Röntgenröhre 12 bzw. des Bildschichtträgers 13 vorgenommen werden soll, werden ausgewählte Motoren 17,19,21,23,25,27 mittels des Steuerpults 32 angesteuert. Die Signale der Lagesensoren 18,20, 22,24,26,28 werden dabei über Verbindungen 33 bis 38 der Regeleinrichtung 29 zugeführt. Die Regeleinrichtung 29 führt dann bei einem vorgegebenen Isozentrum 39 über Verbindungen 40 bis 45 den übrigen Motoren 17,19,21,23, 25,27 Regelgrössen derart zu, dass durch deren Ansteuerung das Isozentrum 39 beibehalten wird. Wenn nun z.B. der Träger 12 um 90° gedreht werden soll, so dass der Zentralstrahl 14 der Röntgenröhre 12 bzw. des Bildschichtträgers 13 eine horizontale Lage einnehmen soll, wird über das Steuerpult 32 der Motor 23 angesteuert, damit der Träger 7 um die Welle 8 geschwenkt wird. Die Regeleinrichtung 29 steuert dann in beschriebener Weise die weiteren Motoren 19,21 und 27 derart nach, dass das voreingestellte Isozentrum 39 bei der Lageänderung der Röntgenröhre bzw. des Bildschichtträgers erhalten bleibt. Die Motoren 19,21 und 27 bewirken, dass der Gelenkarm 6 abgesenkt und geschwenkt und die Säule 4 gedreht wird. Auf entsprechende Weise wird bei anderen gewünschten Bewegungsabläufen auch die Verschiebung des Stativwagens 1 gesteuert und der Träger 7 um die Welle 15 gedreht.

In der Figur 3 ist gezeigt, dass der zweiteilige Gelenkarm 6 durch die beschriebenen Mittel so eingestellt werden kann, dass der zweite Teil 11 des Gelenkarmes 6 in Längsrichtung des Untersuchungstisches 3 zu liegen kommt. Bei dieser Lage des Gelenkarmes 6 bzw. des Trägers 7 mit der Röntgenröhre 12 und dem Bildschichtträger 13 kann in Kombination mit einem weiteren quer zum Untersuchungstisch angebrachten und z.B. deckenaufgehängten Träger 46 mit zugehöriger Röntgenröhre 47 bzw. Bildschichtträger 48 eine Zweiebenenuntersuchung durchgeführt werden. Durch diesen Bewegungsablauf kann das Röntgenuntersuchungsgerät nach der
Erfindung für sowohl Einebene- als auch für Zweiebenenbetrieb verwendet werden und dient daher als Universalstativ.

**0224886**

Patentansprüche

1. Röntgenuntersuchungsgerät mit einer Säule und einer Halterung, die als ein zweiteiliger Gelenkarm ausgebildet ist, die in Bezug auf die Säule in vertikaler Richtung verstellbar ist und einen gebogenen Träger hält, an dessen einem Ende eine Strahlungsquelle und an dessen anderem Ende ein Bildschichtträger aufeinander ausgerichtet befestigt sind, und wobei der Träger um eine erste Welle drehbar gelagert und um eine weitere, horizontale und senkrecht zur ersten Welle am vorderen Ende der Halterung liegende zweite Welle schwenkbar gelagert ist und für jede der Translations- und Drehbewegungen ein Antrieb vorsehbar ist, d a d u r c h   g e k e n n z e i c h n e t, dass die Säule 4 um eine vertikale Achse 5 drehbar gelagert ist und die gemeinsame Schwenkachse 9 des zweiteiligen Gelenkarmes 6 etwa vertikal verläuft.

2. Röntgenuntersuchungsgerät nach Anspruch 1, d a d u r c h   g e k e n n z e i c h n e t, dass der von der Säule 4 ausgehende Teil 10 des Gelenkarmes 6 als Parallelogrammarm ausgebildet ist.

FIG 1

FIG 2

FIG 3

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,Y | EP-A-0 160 749 (SIEMENS AG) * Zusammenfassung; Seite 3, Zeile 16 - Seite 4, Zeile 22; Seite 5, Zeile 21 - Seite 6, Zeile 3; Abbildungen 1-4 * | 1 | A 61 B 6/00 |
| | --- | | |
| Y | US-A-4 223 222 (D.A. GRAY et al.) * Zusammenfassung; Spalte 3, Zeilen 3-40, Zeile 63 - Spalte 4, Zeile 19; Abbildungen 2-5 * | 1 | |
| | --- | | |
| A | FR-A-2 405 695 (SIEMENS AG) * Seite 1, Zeilen 31-38; Seite 3, Zeile 21 - Seite 6, Zeile 20; Abbildungen 2,3 * | 1,2 | |
| | --- | | RECHERCHIERTE SACHGEBIETE (Int Cl 4) |
| A | US-A-2 287 577 (R.J. STAVA) * Seite 1, rechte Spalte, Zeilen 15-52; Abbildungen 1,8 * | 1,2 | A 61 B |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 02-03-1987 | RIEB K.D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82